# EUROPEAN PATENT SPECIFICATION

(11) **EP 1 867 345 B1**
(45) Date of publication and mention of the grant of the patent: **29.07.2009**
(21) Application number: 06010770.3
(22) Date of filing: 24.05.2006
(51) Int. Cl.: A61L 2/18, A61L 2/20, A61L 101/10

(54) **Disinfectant appliance with a nanometer disinfectant function**
Gerät zum Desinfizieren mit einer Nanometer-Desinfizierungsfunktion
Appareil pour désinfection avec une fonction désinfectant nanométrique

(43) Date of publication of application: 19.12.2007
(73) Proprietor: ROSACE INTERNATIONAL CO., LTD., T'ai pei (TW)
(72) Inventor: Tseng, Kuang-Tai, Yonghe City Taipei Hsien (TW); Chen, Kuo-Kang, Taipei (TW)
(74) Representative: Casalonga, Axel

(56) References cited:
- EP-A1- 1 287 834
- EP-A1- 1 302 250
- EP-A2- 0 978 290
- WO-A-02/48054
- CN-Y- 2 623 683
- JP-A- 6 233 805
- JP-A- 11 036 394
- JP-A- 2003 052 556

## Description

The present invention relates to a disinfectant appliance, especially to a disinfectant appliance that provides a nanometer disinfectant function.

Foods, containers for foods, utensils and medical instruments must be washed to remove foreign matter and sterilized to kill bacteria, viruses and other microscopic contamination. The conventional way to sterilize objects uses chemicals. However, the chemicals must be toxic to kill bacteria and viruses. If the chemicals are not rinsed completely off the objects, the residual chemicals may also injure the human body. However, chemicals rinsed into the sewer system pollute the environment. To prevent pollution, wastewater must be collected and treated or filtered. Therefore, the conventional way to sterilize objects is not safe and is not convenient.

A safe and a sufficient way to sterilize objects are to emulsify water. Emulsifying water has multiple steps. First, a large amount of gas is compressed into the water. Then pressure on the water is released to release the gas. The released gas forms innumerable tiny bubbles in almost nanometer size to bring out the dirt, bacteria and viruses. Emulsification pressure-releasing devices were developed to provide emulsified water. The more gas compressed into the water, the more pressure is needed. To bear a greater pressure level, the size of the conventional emulsification pressure-releasing device is larger. The large emulsification pressure-releasing device is not easy to be moved or set up.

EP 0 978 290 discloses a cleaning faucet device comprising a sink, a water supplying head, a proximity sensor disposed in the vicinity of the discharge port, an ozone water source configured as an ozone-water producing apparatus for producing ozone-water using a quality improved water supplied by a branch line from a pipe connected to pre-cleaning water source, a solenoid valve coupled to the ozone-water source, a needle valve coupled to the solenoid valve, and a pipe coupled to the needle valve and to the discharge port of the water supplying head. The function of the needle valve included in pipe is to adjust the setting flow rate of ozone-water. The function of the solenoid valve is to allow circulating the ozone-water for improving or keeping the concentration of the ozone-water by making use of the circulating pressure.

JP 2003/052556 discloses a an automatic hand washing system with ozone water comprising a faucet, a hand washing sink, a hand washing detecting sensor generating a detecting signal, and an ozone water manufacturing unit which obtains ozone water by dissolving ozone gas from an ozone generating unit with supply water fed through an electromagnetic valve. The function of the faucet is to discharge ozone-water.

JP 2006/233805 discloses a hand washing disinfector unit comprising an ozone gas generator; mixing means for mixing the ozone gas produced by the ozone gas generator with feed water such as service water into ozone water; a reaction-dissolution tank storing the ozone water generated by the mixing means; a faucet discharging the ozone water; and a wash section.

EP 1 287 834 discloses an in-line system for ozone sanitation, comprising a termination of line, which can be capped with a valve or other fitting facilitating the application of ozonated solution to the product or service being sanitized; an ozone generator fed by a high purity O₂ source; an element connected to the ozone generator; a proxair ejector injecting ozone into feed water supplied from a storage tank by pump for filtering and O₃ dissolution through a filter to the proxair ejector; a gas-liquid phase separator; a process pump connected by line to the gas-liquid separator; and line terminating at valve.

None of the available prior art documents mentions or suggests a disinfection applicance with a nanometer disinfectant function comprising a pressure-releasing device according to the present invention, see below, for emulsifying pressurized liquid to release innumerably tiny bubbles in almost nanometer size.

To overcome the shortcomings, the present invention provides a disinfectant appliance that provides nanometer disinfectant function to mitigate or obviate the aforementioned problems.

The main objective of the present invention is to provide a disinfectant appliance that provides nanometer disinfectant function. The disinfectant appliance with a nanometer disinfectant function has a gas generator and an emulsification pressure-releasing device. The emulsification pressure-releasing device has a pressurizing device, a pressure-equalizing device and the pressure-releasing device to pressurize water and gas and to emulsify the pressurized liquid. The emulsified liquid can take out the dirt bacteria and viruses to completely clean the hands and the things.

Accordingly and as claimed, there is provided a disinfectant appliance with a nanometer disinfectant function comprising a faucet, a gas generator generating gas, an emulsification pressure-releasing device connected to the gas generator and comprising a pressurizing device connecting to the gas generator, a switch connecting to the faucet and the pressurizing device to selectively switch on the faucet and the pressurizing device, a pressure-equalizing device connecting to the pressurizing device, and at least one pressure-releasing device connecting to the pressure-equaling device; a connecting tube connecting to each one of the at least one pressure-releasing device, and a hollow shell including an open top, a close bottom, an inner wall, a sink formed in and communicating with the open top of the shell and having a bottom wall, a sidewall, a bottom outlet formed through the bottom wall of the shell, and a side outlet formed through the bottom wall of the shell.

According to the invention, the hollow shell further includes a water reservoir formed in the shell and comprising a bottom, a sidewall, a sensor mounted in the water reservoir, an entering switch attached to the sidewall of the water reservoir and connecting to the sensor, and an aperture formed through the sidewall of the water reservoir, and a placing tank formed in the close bottom, wherein the gas generator and the emulsification pressure-releasing device are mounted in the placing tank.

Further according to the invention, each one of the at least one pressure-releasing device comprises a communicating tube connecting to the water reservoir and the gas generator, a branch tube connecting to and communicating with the communicating tube and having an inner diameter, a top, a bottom, and a threaded hole formed through the top of the branch tube, an exhausting tube formed transversely on and communicating with the branch tube, a bottom collar mounted in the bottom of the branch tube and having a central hole; a threaded rod mounted in the threaded hole in the branch tube and having a bottom, and a base disc mounted on the bottom of the , threaded rod, fitting in the branch tube and being located above the exhausting tube, and a main floating disc mounted floatably in the branch tube, being between the bottom of the branch tube and the base disc of the threaded rod and having an outer diameter being smaller than the inner diameter of the branch tube; a bottom surface facing the bottom of the branch tube, and multiple detents formed in the bottom surface of the main floating disc.

Other objectives, advantages and novel features of the invention will become more apparent from the following detailed description when taken in conjunction with the accompanying drawings, in which:
Fig. 1 is a side view in partial section of a disinfectant appliance with a nanometer disinfectant function in accordance with the present invention;
Fig. 2 is a perspective view of an emulsification pressure-releasing device in the disinfectant appliance in Fig. 1;
Fig. 3 is a side view in partial section of the emulsification pressure-releasing device in Fig. 2;
Fig. 4 is a top view of a main floating disc in the emulsification pressure-releasing device in Fig. 2;
Fig. 5 is a cross-sectional side view of the main floating disc in the emulsification pressure-releasing device in Fig. 2;
Fig. 6 is a top view of a secondary floating disc in the emulsification pressure-releasing device in Fig. 2;
Fig. 7 is a cross-sectional side view of the secondary floating disc in the emulsification pressure-releasing device in Fig. 2; and
Fig. 8 is a side view in partial section of another embodiment of the emulsification pressure-releasing device in the disinfectant appliance in Fig. 1.

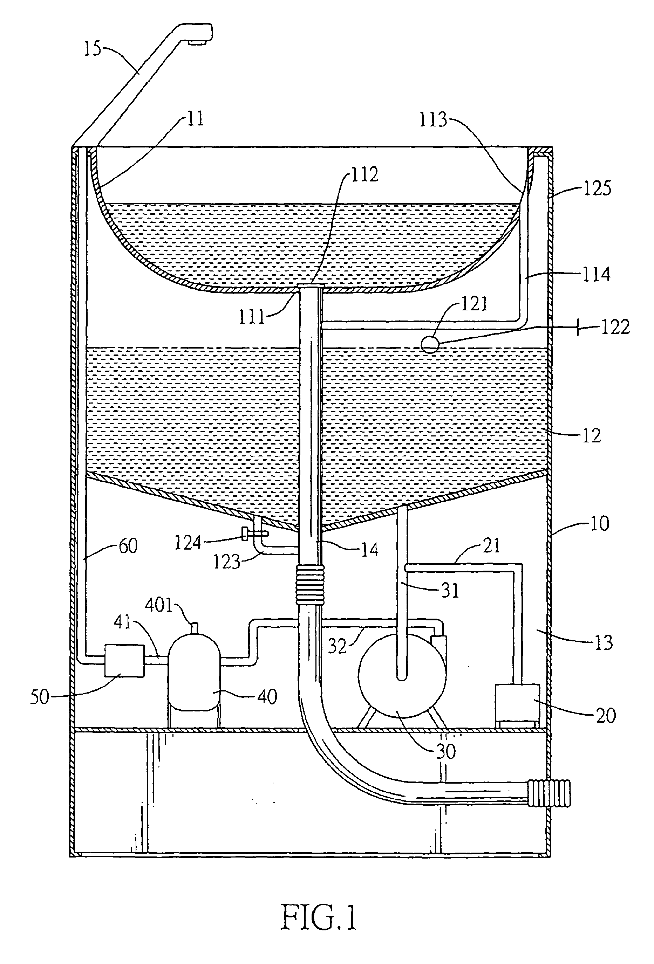

With reference to Fig. 1, a disinfectant appliance with a nanometer disinfectant function in accordance with the present invention comprises a shell (10), a gas generator (20), an emulsification pressure-releasing device and a connecting tube (60). To be used more conveniently, multiple disinfectant appliances of the present invention can be applied together.

The shell (10) is hollow and has an open top, a closed bottom, an inner wall, a sink (11), a plug (112), a water reservoir (12), a placing tank (13), a drain (14), a spillway (114), an adapting tube (123) and a faucet (15).

The sink (11) is formed in and communicates with the open top of the shell (10) and has a bottom wall, a sidewall, a bottom outlet (111) and a side outlet (113). The bottom outlet (111) is formed through the bottom wall of the shell (10). The side outlet (113) is formed through the sidewall of the shell (10). When the water in the sink (11) is too much and the water level reaches the side outlet (113), the water flow out of the sink (11) through the side outlet (113). The plug (112) selectively seals the bottom outlet (111) to keep the water in the sink (11) from flowing out of the sink (11) through the bottom outlet (111).

The water reservoir (12) is formed in the shell (10) and comprises a bottom, a sidewall, a sensor (121), an entering switch (122) and an aperture (125). The sensor (121) is mounted in the water reservoir (12) to detect the amount of the water in the water reservoir (12). The entering switch (122) is attached to the sidewall of the water reservoir (12) and connects to the sensor (121) to control the water flowing into the water reservoir (12) by the detection of the sensor (121). The aperture (125) is formed through the sidewall of the water reservoir (12) to equalize the pressure in the water reservoir (12) and outside of the water reservoir (12).

The placing tank (13) is formed in the closed bottom of the shell (10). The drain (14) connects to and communicates with the bottom outlet (111) in the sink (11), extends through the water reservoir (12) and extends out of the placing tank (13).

The spillway (114) has two ends respectively connected to and communicating with the side outlet (113) in the sink (11) and the drain (14). When the water flow out of the sink (11) from the side outlet (113), the water flow through the spillway (114) into the drain (14).

The adapting tube (123) connects to and communicates with the water reservoir (12) and the drain (14) and has an exhausting switch (124) mounted on the adapting tube (123) to selectively allow the water flowing through the adapting tube (123).

The faucet (15) is mounted on the sink (11).

The gas generator (20) is mounted in the placing tank (13) and comprises an air tube (21) extending out of the gas generator (20) to deliver the gas out of the gas generator (20). The gas generator (20) can generate gases such as ozone.

The emulsification pressure-releasing device is mounted in the placing tank (13) and comprises a pressurizing device (30), a switch (not shown), a water tube (31), a first delivering tube (32), a pressure-equalizing device (40), second delivering tube (41) and at least one pressure-releasing device (50).

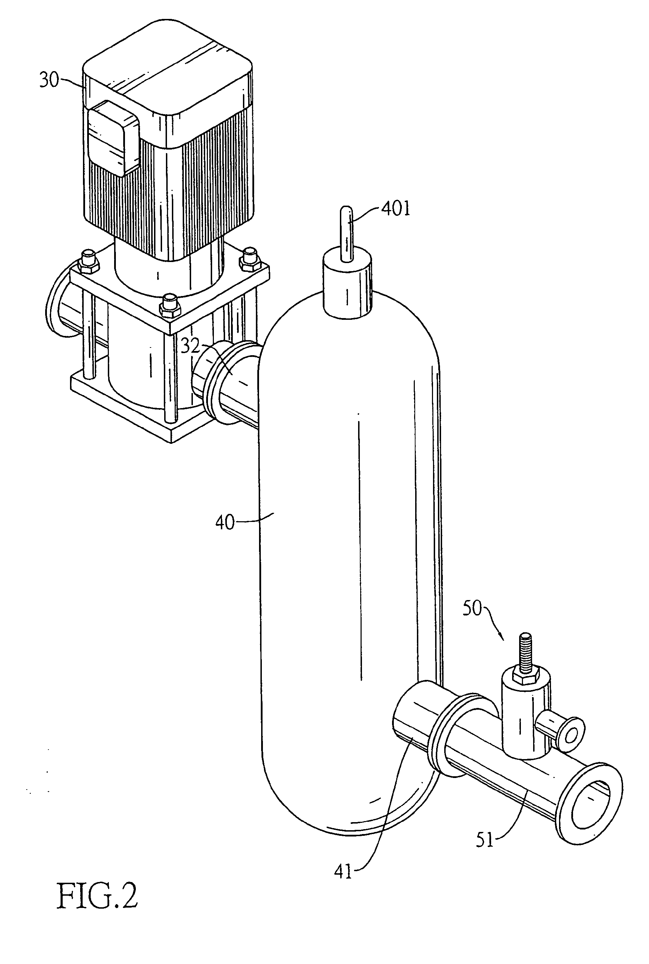

With further reference to Fig. 2, the pressurizing device (30) is mounted in the placing tank (13) and connects to and communicates with the air tube (21) to allow the gas pumping into the pressurizing device (30).

The switch is mounted on the sink (11) and connects to the faucet (15) and the pressurizing device (30) to selectively switch automatically or manually on the faucet (15) and the pressurizing device (30). The switch can be a photo-sensor.

The water tube (31) connects to and communicates with the water reservoir (12) and the pressurizing device (30) to allow the water in the water reservoir (12) pumping into the pressurizing device (30). The pressurizing device (30) pressurizes the gas and the water to dissolve the gas in the water and to become the pressurizing liquid. The first delivering tube (32) connects to and communicates with the pressurizing device (30) to allow the pressurized liquid flowing out of the pressurizing device (30).

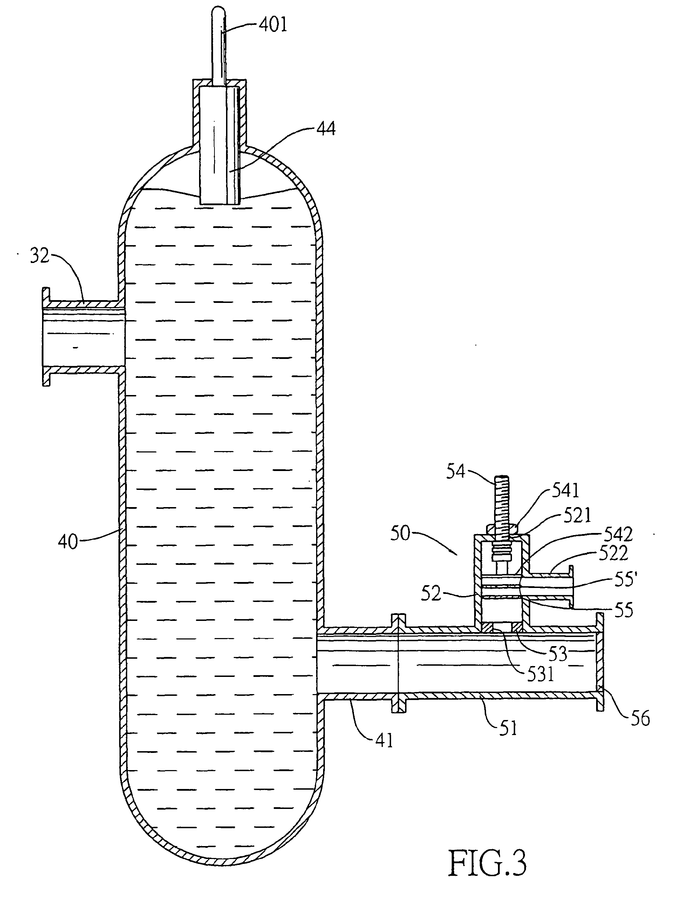

With further reference to Fig. 3, the pressure-equalizing device (40) connects to and communicates with the first delivering tube (32) to allow the pressurizing liquid flowing into the pressure-equalizing device (40) and comprises an adjusting button (401) and a float (44). The adjusting button (401) is mounted in and extends out of the pressure-equalizing device (40). The float (44) is mounted in the pressure-equalizing device (40) and selectively pushes the adjusting button (401). The float (44) is floated on the water level of the pressurized liquid in the pressure-equalizing device (40). When the pressurized liquid in the pressure-equalizing device (40) achieves a desired level to make the float (44) push the adjusting button (401), the adjusting button (401) is pushed to releasing surplus gas.

The at least one pressure-releasing device (50) is mounted in the placing tank (13) and connects to and communicates with the pressure-equalizing device (40) to allow the pressurized liquid flowing into the pressure-releasing device (50). Each pressure-releasing device (50) comprises a communicating tube (51), a branch tube (52), an exhausting tube (522), a bottom collar (53), a threaded rod (54), a main floating disc (55), at least one secondary floating disc (55') and a sealing board (56).

The communicating tube (51) has a front end and a rear end. The front end of the communicating tube (51) connects to and communicates with the second delivering tube (41).

The branch tube (52) connects to and communicates with the communicating tube (51) and has an inner diameter, a top, a bottom and a threaded hole (521). The threaded hole (521) is formed through the top of the branch tube (52).

The exhausting tube (522) is formed transversely on and communicates with the branch tube (52).

The bottom collar (53) is mounted in the bottom of the branch tube (52) and has a central hole (531).

The threaded rod (54) is mounted in the threaded hole (521) in the branch tube (52) and has a bottom and a base disc (542). The base disc (542) is mounted on the bottom of the threaded rod (54), fits in the branch tube (52) and is located above the exhausting tube (522).

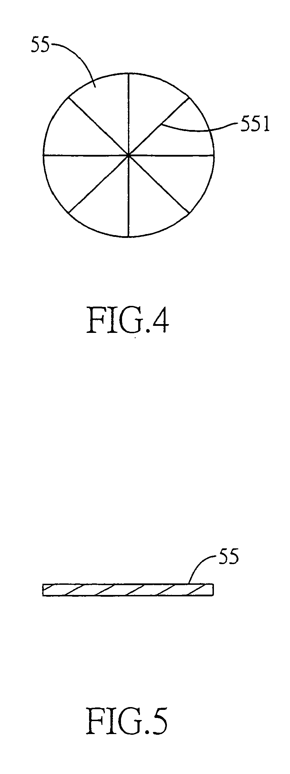

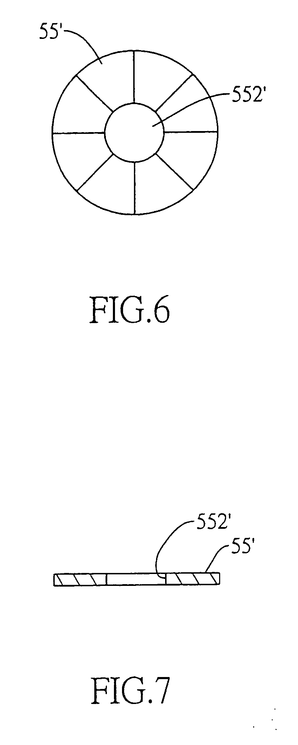

With further reference to Figs. 4 to 7, the main and secondary floating discs (55, 55') are mounted floatablly in the branch tube (52) and are between the bottom of the branch tube (52) and the base disc (542) on the threaded rod (54). Each floating disc (55, 55') has an outer diameter, a bottom surface and multiple detents (551). The outer diameter of the floating discs (55, 55') are smaller than the inner diameter of the branch tube (52) to allow the floating discs (55, 55') floating in the branch tube (52). The bottom surfaces of the floating discs (55, 55') face to the bottom of the branch tube (52). The detents (551) are formed in the bottom surface of the floating discs (55, 55') by electrically discharging process and is sized as few micrometers. The secondary floating disc (55') has a central hole (552') and is below the main floating disc (55).

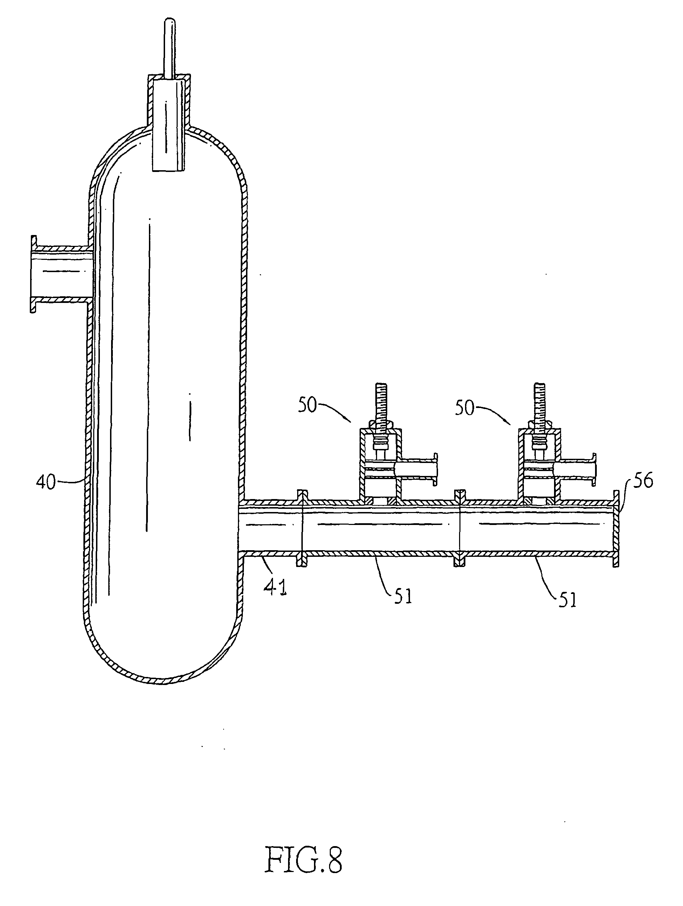

With further reference to Fig. 8, multiple pressure-releasing device (50) connect serially. The front end of the communicating tube (51) of each pressure-releasing device (50) connects to and communicates with the fore communicating tube (51) of the pressure-releasing device (50). The front end of the communicating tube (51) of the first pressure-releasing device (50) connects to and communicates with the second delivering tube (41). The sealing board (56) seals the rear end of the last communicating tube (51).

The connecting tube (60) respectively connects to and communicates with the exhausting tube (522) of each pressure-releasing device (50) and connects to and communicates with the faucet (15) on the sink (11).

The water fills in the water reservoir (12) and the gas generator (20) generates the ozone. When the faucet (15) is switched on automatically or manually, the pressurizing device (30) pumps the water and the gas into the pressurizing device (30) to become pressurized liquid. The pressurized liquid flows through the pressure-equalizing device (40) and flows into the pressure-releasing device (50). The pressurized liquid flows along the detents in the floating discs (55, 55') and flows through the gap between the floating discs (55, 55') and the branch tube (52). Then the pressurized liquid flows out of the exhausting tube (522) to be emulsified to release innumerable tiny bubbles in almost nanometer size. The emulsified liquid flows through the connecting tube (60) and flows out of the faucet (15) to clean the hands and the things. The tiny bubbles take out the dirt, bacteria and viruses to completely clean the hands and the things.

Even though numerous characteristics and advantages of the present invention have been set forth in the foregoing description, together with details of the structure and features of the invention, the disclosure is illustrative only. Changes may be made in the details, especially in matters of shape, size, and arrangement of parts within the principles of the invention to the full extent indicated by the broad general meaning of the terms in which the appended claims are expressed.

## Claims

1. A disinfectant appliance with a nanometer disinfectant function comprising:
a faucet (15),
a gas generator (20) generating gas,
an emulsification pressure-releasing device connected to the gas generator (20) and comprising
a pressurizing device (30) connecting to the gas generator (20),
a switch connecting to the faucet (15) and the pressurizing device (30) to selectively switch on the faucet (15) and the pressurizing device (30),
a pressure-equalizing device (40) connecting to the pressurizing device (30), and
at least one pressure-releasing device (50) connecting to the pressure-equalizing device (40);
a connecting tube (60) connecting to each one of the at least one pressure-releasing device (50), and
a hollow shell (10) including
an open top,
a close bottom,
an inner wall,
a sink (11) formed in and communicating with the open top of the shell (10) and having
a bottom wall,
a sidewall,
a bottom outlet (111) formed through the bottom wall of the shell (10), and
a side outlet (113) formed through the bottom wall of the shell (10),
**characterized in that** the hollow shell (10) further includes a water reservoir (12) formed in the shell (10) and comprising
a bottom,
a sidewall,
a sensor (121) mounted in the water reservoir (12),
an entering switch (122) attached to the sidewall of the water reservoir (12) and connecting to the sensor (121), and
an aperture (125) formed through the sidewall of the water reservoir (12), and
a placing tank (13) formed in the close bottom, wherein the gas generator (20) and the emulsification pressure-releasing device are mounted in the placing tank (13),
and **in that** each one of the at least one pressure-releasing device (50) comprises
a communicating tube (51) connecting to the water reservoir (12) and the gas generator (20).
a branch tube (52) connecting to and communicating with the communicating tube (51) and having
an inner diameter,
a top,
a bottom, and
a threaded hole (521) formed through the top of the branch tube (52).
an exhausting tube (522) formed transversely on and communicating with the branch tube (52),
a bottom collar (53) mounted in the bottom of the branch tube (52) and having a central hole (531);
a threaded rod (54) mounted in the threaded hole (521) in the branch tube (52) and having
a bottom, and
a base disc (542) mounted on the bottom of the threaded rod (54), fitting in the branch tube (52) and being located above the exhausting tube (522), and
a main floating disc (55) mounted floatably in the branch tube (52), being between the bottom of the branch tube (52) and the base disc (542) of the threaded rod (54) and having
an outer diameter being smaller than the inner diameter of the branch tube (52);
a bottom surface facing the bottom of the branch tube (52), and multiple detents (551) formed in the bottom surface of the main floating disc (55).

2. The disinfectant appliance with a nanometer disinfectant function as claimed in claim 1, wherein the switch is a photo-sensor.

3. The disinfectant appliance with a nanometer disinfectant function as claimed in claim 1, wherein the shell (10) has
a plug (112) attached to the bottom outlet (111) of the sink (11) for selectively sealing the bottom outlet (111),
a drain (14) connecting to and communicating with the bottom outlet (111) in the sink (11), extending through the water reservoir (12) and extending out of the placing tank (13),
a spillway (114) connecting to and communicating with the side outlet (113) in the sink (11) and the drain (14), and
an adapting tube (123) connecting to and communicating with the water reservoir (12) and the drain (14) and having an exhausting switch (124) mounted on the adapting tube (123).

4. The disinfectant appliance with a nanometer disinfectant function as claimed in claim 1, wherein each one of the at least one pressure-releasing device (50) comprises
at least one secondary floating disc (55') mounted floatably in the branch tube (52), being between the bottom of the branch tube (52) and the base disc (542) of the threaded rod (54), being below the main floating disc (55) and having
an outer diameter being smaller than the inner diameter of the branch tube (52);
a bottom surface facing to the bottom of the branch tube (52), and
multiple detents (551) formed in the bottom surface of the secondary floating disc (55').

## Patentansprüche

1. Eine Desinfektionsvorrichtung mit einer Nanometerdesinfektionsfunktion, umfassend:
einen Zapfhahn (15),
eine Gas erzeugende Gaserzeugungseinrichtung (20), eine Emulgierungs- und Druckablassvorrichtung, die mit der Gaserzeugungseinrichtung (20) verbunden ist und folgendes umfasst:
eine Druckbeaufschlagungseinrichtung (30), die mit der Gaserzeugungseinrichtung (20) verbunden ist,
eine Schalteinrichtung, die mit dem Zapfhahn (15) und der Druckbeaufschlagungseinrichtung (30) verbunden ist, um den Zapfhahn (15) und
die Druckbeaufschlagungsvorrichtung (30) selektiv zu schalten,
eine Druckausgleichseinrichtung (40), die mit der Druckbeaufschlagungseinrichtung (30) verbunden ist, und
mindestens eine Druckablasseinrichtung (50), die mit der Druckausgleichseinrichtung (40) verbunden ist;
eine Verbindungsrohrleitung (60), die mit einer jeweiligen der mindestens einen Druckablassvorrichtung (50) verbunden ist, und
eine hohle Ummantelung (10) mit folgendem:
einer offenen Oberseite,
einer geschlossenen Unterseite,
einer Innenwand,
einem Ausgussbecken (11), das in der offenen
Oberseite der Ummantelung (10) ausgebildet ist und mit der offenen Seite kommuniziert und das folgendes aufweist:
eine unterseitige Wand,
eine Seitenwand,
einen unterseitigen Auslass (111), der durch die unterseitige Wand der Ummantelung (10) hindurch ausgebildet ist, und
einen Seitenauslass (113), der durch die unterseitige Wand der Ummantelung (10) ausgebildet ist,
**dadurch gekennzeichnet, dass** die hohle Ummantelung (10) ferner einen Wassertank (12) umfasst, der in der Ummantelung (10) ausgebildet ist und
der folgendes umfasst:
eine Unterseite,
eine Seitenwand,
einen in dem Wassertank (12) montierten Sensor (121),
einen Einlassschalter (122), der an der Seitenwand des Wassertanks (12) befestigt ist und der mit dem Sensor 121 verbunden ist, und
eine Öffnung (125), die durch die Seitenwand des Wassertanks (12) ausgebildet ist, und
einen Unterbringungsbehälter (13), der in der geschlossenen Unterseite ausgebildet ist, wobei die Gaserzeugungseinrichtung (20) und die Emulgierungs- und Druckablasseinrichtung in dem Unterbringungsbehälter (13) montiert sind,
und **dadurch**, dass eine jeweilige der mindestens einen Druckablassvorrichtung (50) folgendes umfasst:
eine Kommunikationsrohrleitung (51), die mit dem Wassertank (12) und der Gaserzeugungseinrichtung (20) verbunden ist,
eine Abzweigrohrleitung (52), die verbunden ist und kommuniziert mit der Kommunikationsrohrleitung (51) und die folgendes aufweist:
einen inneren Durchmesser,
eine Oberseite,
eine Unterseite und
ein mit einem Gewinde versehenes Loch (521), das durch die Oberseite der Abzweigungsrohrleitung (52) ausgebildet ist,
ein Ablassrohr (522), das transversal auf und kommunizierend mit der Abzweigrohrleitung (52) ausgebildet ist,
eine unterseitige Manschette (53), die in der Unterseite der Abzweigrohrleitung (52) montiert ist und die
ein mittiges Loch (531) aufweist; eine mit einem Gewinde versehene Stange (54), die in dem mit dem Gewinde versehenen Loch (521) in der Abzweigrohrleitung (52) montiert ist und die folgendes aufweist:
eine Unterseite und
eine Basisscheibe (542), die auf der Unterseite der mit einem Gewinde versehenen Stange (54) montiert ist, die in die Abzweigrohrleitung (52) eingepasst ist und die über der Ablassrohrleitung (522) angeordnet ist, und
eine Hauptschwimmerscheibe (55), die schwebend in der Abzweigungsrohrleitung (52) montiert ist, die zwischen der Unterseite der Abzweigungsrohrleitung (52) und der Basisscheibe (542) der mit einem Gewinde versehenen Stange (54) ist, und die folgendes aufweist:
einen äußeren Durchmesser, der kleiner ist als der innere Durchmesser der Abzweigungsrohrleitung (52),
eine untere Oberfläche, die der Unterseite der Abzweigungsrohrleitung (52) gegenüberliegt, und
mehrere Rastungen (551), die in der unterseitigen Oberfläche der Hauptschwimmerscheibe (55) ausgebildet sind.

2. Die Desinfektionsvorrichtung mit einer Nanometerdesinfektionsfunktion nach Anspruch 1, wobei der Schalter ein Photosensor ist.

3. Die Desinfektionsvorrichtung mit einer Nanometerdesinfektionsfunktion nach Anspruch 1, wobei die Ummantelung (10) folgendes aufweist:
einen Absperrhahn (112), der an dem unterseitigen Auslass (111) des Ausgussbeckens (11) zum selektiven Verschließen des unterseitigen Auslasses (111) befestigt ist,
ein Abfluss (14), der verbunden ist und kommuniziert mit dem unterseitigen Auslass (111) in dem Ausgussbecken (11), der sich durch den Wassertank (12) erstreckt und der sich aus dem Unterbringungsbehälter (13) hinaus erstreckt,
einen Überlauf (114), der verbunden ist und kommuniziert mit dem Seitenauslass (113) in dem Ausgussbecken (11) und dem Abfluss (14) und
eine Anpassrohrleitung (123), die verbunden ist und
kommuniziert mit dem Wassertank (12) und dem Abfluss (14) und die einen an der Anpassrohrleitung (123) montierten Ablassschalter (124) aufweist.

4. Die Desinfektionsvorrichtung mit einer Nanometerdesinfektionsfunktion nach Anspruch 1, wobei eine jeweilige der mindestens einen Druckablassvorrichtung (50) folgendes umfasst:
mindestens eine Sekundärschwimmerscheibe (55'), die schwebend in der Abzweigungsrohrleitung (52) montiert ist, die zwischen der Unterseite der Abzweigungsrohrleitung (52) und der Basisscheibe (542) der mit einem Gewinde versehenen Stange (54) ist, die unterhalb der Hauptschwimmerscheibe (55) ist und die folgendes aufweist:
einen äußeren Durchmesser, der kleiner ist als der innere Durchmesser der Abzweigungsrohrleitung (52),
eine unterseitige Oberfläche, die der Unterseite der Abzweigungsrohrleitung (52) gegenüberliegt, und
mehrere Rasterungen (551), die in der unterseitigen Oberfläche der Sekundärschwimmerscheibe (55') ausgebildet sind.

## Revendications

1. Appareil de désinfection ayant une fonction de désinfectant nanométrique comprenant :
un robinet (15),
un générateur de gaz (20) fournissant du gaz,
un dispositif de libération de pression d'émulsification connecté au générateur de gaz (20) et comprenant :
un dispositif de pressurisation (30) se connectant au générateur de gaz (20),
un interrupteur se connectant au robinet (15) et au dispositif de pressurisation (30) pour déclencher de manière sélective le robinet (15) et le dispositif de pressurisation (30),
un dispositif d'égalisation de pression (40) se connectant au dispositif de pressurisation (30), et
au moins un dispositif de libération de pression (50) se connectant au dispositif d'égalisation de pression (40) ;
un tube de connexion (60) se connectant à chacun des dispositifs de libération de pression (50), et
une coque creuse (10) comprenant :
une partie supérieure ouverte,
une partie inférieure fermée,
une paroi intérieure,
un évier (11) formé dans et communiquant avec la partie supérieure ouverte de la coque (10) et comportant :
une paroi inférieure,
une paroi latérale,
une sortie de fond (111) formée dans la paroi inférieure de la coque (10), et
une sortie latérale (113) formée dans la paroi inférieure de la coque (10),
**caractérisé en ce que** la coque creuse (10) comprend en outre un réservoir d'eau (12) formé dans la coque (10) et comprenant :
un fond,
une paroi latérale,
un capteur (121) monté dans le réservoir d'eau (12),
un interrupteur entrant (122) fixé à la paroi latérale du réservoir d'eau (12) et se connectant au capteur (121), et
une ouverture (125) formée dans la paroi latérale du réservoir d'eau (12), et
un compartiment de montage (13) formé dans la partie inférieure fermée, le générateur de gaz (20) et le dispositif de libération de pression d'émulsification étant montés dans le compartiment de montage (13),
et **en ce que** chacun des dispositifs de libération de pression (50) comprend :
un tube de communication (51) se connectant au réservoir d'eau (12) et au générateur de gaz (20),
un tube de dérivation (52) se connectant à et communiquant avec le tube de communication (51) et ayant :
un diamètre intérieur,
une partie supérieure,
une partie inférieure, et
un trou fileté (521) formé dans la partie supérieure du tube de dérivation (52),
un tube d'échappement (522) formé de manière transversale sur et communiquant avec le tube de dérivation (52),
un collier inférieur (53) monté dans la partie inférieure du tube de dérivation (52) et comportant un trou central (531) ;
une tige filetée (54) montée dans le trou fileté (521) du tube de dérivation (52) et comportant :
une partie inférieure, et
un disque de base (542) monté sur la partie inférieure de la tige filetée (54), se logeant dans le tube de dérivation (52) et situé au-dessus du tube d'échappement (522), et
un disque flottant principal (55) monté de manière flottante dans le tube de dérivation (52), situé entre la partie inférieure du tube de dérivation (52) et le disque de base (542) de la tige filetée (54) et ayant :
un diamètre extérieur inférieur au diamètre intérieur du tube de dérivation (52) ;
une surface inférieure faisant face à la partie inférieure du tube de dérivation (52), et
plusieurs crans (551) formés dans la surface inférieure du disque flottant principal (55).

2. Appareil de désinfection ayant une fonction de désinfectant nanométrique selon la revendication 1, dans lequel l'interrupteur est un capteur optique.

3. Appareil de désinfection ayant une fonction de désinfectant nanométrique selon la revendication 1, dans lequel la coque (10) comporte :
un bouchon (112) fixé à la sortie de fond (111) de l'évier (11) pour boucher de manière sélective la sortie de fond (111),
un drain (14) se connectant à et communiquant avec la sortie de fond (111) de l'évier (11), s'étendant à travers le réservoir d'eau (12) et s'étendant hors du compartiment de montage (13),
un évacuateur (114) se connectant à et communiquant avec la sortie latérale (113) de l'évier (11) et le drain (14), et
un tube d'adaptation (123) se connectant à et communiquant avec le réservoir d'eau (12) et le drain (14) et comportant un interrupteur de vidange (124) monté sur le tube d'adaptation (123).

4. Appareil de désinfection ayant une fonction de désinfectant nanométrique selon la revendication 1, dans lequel chacun des dispositifs de libération de pression (50) comprend :
au moins un disque flottant secondaire (55') monté de manière flottante dans le tube de dérivation (52), situé entre la partie inférieure du tube de dérivation (52) et le disque de base (542) de la tige filetée (54), sous le disque flottant principal (55) et ayant :
un diamètre extérieur inférieur au diamètre intérieur du tube de dérivation (52) ;
une surface inférieure faisant face à la partie inférieure du tube de dérivation (52), et
plusieurs crans (551) formés dans la surface inférieure du disque flottant secondaire (55').
